# EUROPEAN PATENT APPLICATION

(11) **EP 1 241 248 A1**
(43) Date of publication of application: **18.09.2002**
(21) Application number: 02251422.8
(22) Date of filing: 28.02.2002
(51) Int. Cl.: C12N 1/20, C12N 1/38, C12P 1/04

(54) **Method of proliferating a microorganism capable of degrading a hard-to-degrade organic compound and use thereof**

(30) Priority: 13.03.2001 JP 2001070857
(71) Applicant: Kabushiki Kaisha Toshiba, Tokyo 105-8001 (JP)
(72) Inventor: Ito, Keiko, c/o Kabushiki Kaisha Toshiba, ITD, Tokyo 105-8001 (JP); Imada,Toshihiro, c/o Kabushiki Kaisha Toshiba, ITD, Tokyo 105-8001 (JP); Ikeda, Michio, c/o Kabushiki Kaisha Toshiba, ITD, Tokyo 105-8001 (JP)
(74) Representative: Andrews, Timothy Stephen

(57) **Abstract**

The present invention provides a method of proliferating a microorganism capable of degrading a hard-to-degrade organic compound, comprising proliferating at least one microorganism capable of degrading a hard-to-degrade organic compound selected from the group consisting of *Janibacter* genus, *Pseudomonas* genus, Rhodococcus genus, *Desulfomonile* genus, *Alcaligenes* genus, *Bacillus* genus, *Streptococcus* genus, *Acinetobacter* genus, *Achromobacter* genus, Paracoccus genus, Rhodobacter genus, *Rhodobacterium* genus, *Methylosinus* genus, *Mycobacterium* genus, Nitrosomonas genus, *Corynebacterium* genus, and Methanotrophs, in a culture medium containing both a substance capable of inducing the degradation capability of the microorganism and Fe ions, under inorganic conditions. The present invention also provides a method of degrading a hard-to-degrade organic compound by using a microorganism capable of degrading the hard-to-degrade organic compound, comprising a step of controlling the degradation capability of the microorganism by adjusting the concentration of Fe ions in the culture medium.

## Description

The present invention relates to a method of proliferating a microorganism capable of degrading a hard-to-degrade organic compound. The present invention also relates to a method of degrading a hard-to-degrade organic compound by using the microorganism.

In recent years, drainage, ground water, soil and so on have been polluted with volatile organic chlorine compounds such as trichloroethylene. Such a problem has been caused throughout the world.

Up to now, to treat such volatile organic chlorine compounds dissolved in drainage, ground water, etc. at low cost, activated carbon has been used. However, in the case of the treatment of a liquid containing much floating matter, pores of activated carbon are easily blocked. Also, when a low concentration of a compound is treated with activated carbon, the treatment cost becomes high. Furthermore, when activated carbon is used for treatment, a volatile organic chlorine compound adsorbed to the activated carbon must be finally treated by some means. If the organic chlorine compound is toxic, the treatment thereof involves danger. Additionally, when a general combustion method is employed for treating such a toxic organic chlorine compound, dioxins may be generated, and thus it is said that intensive control of the combustion is necessary. However, specific and expensive equipment is required for such intensive control. Therefore, it is considered that the treatment cost of a compound adsorbed to activated carbon increases in the future.

Lately, as a new treatment method whose environmental load is light, a treatment method using a microorganism capable of degrading organic chlorine compounds (i.e., bioremediation) has been proposed. Examples of the bioremediation hitherto reported are as follows: a method of supplying a nutrient source and oxygen to polluted soil in order to increase the degradation activity of a microorganism that lives therein; a method of injecting a microorganism having enhanced degradation capability into polluted soil; and a method of treating polluted drainage or ground water in a reaction vessel packed with a microorganism capable of degrading the pollutant. All of these methods have advantage over conventional methods in that a pollutant can be degraded without causing secondary pollution.

However, such microbial degradation of a pollutant takes a lot of time, which is the greatest drawback. Under the circumstances, there has been a desire to develop a method of enhancing the degradation capability of the microorganism. However, it is considered difficult to artificially control the degradation capability of the microorganism.
No control factors have been found hitherto except for temperature.

The present invention has been made to solve the aforementioned problems of the prior art. An object of the present invention is to increase the degradation capability of the microorganism to be used in a method of degrading a hard-to-degrade organic compound. Further object of the present invention is to control the degradation capability of the microorganism in a method of degrading a hard-to-degrade organic compound.

According to an aspect of the present invention, there is provided a method of proliferating a microorganism capable of degrading a hard-to-degrade organic compound, comprising a step of proliferating at least one microorganism capable of degrading a hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, in a culture medium containing both a substance capable of inducing the degradation capability of the microorganism and Fe ions, under inorganic conditions.

According to another aspect of the present invention, there is provided a method of degrading a hard-to-degrade organic compound by using a microorganism capable of degrading the hard-to-degrade organic compound, comprising a step of controlling the degradation capability of at least one microorganism capable of degrading the hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, by adjusting the concentration of Fe ions in a culture medium.

According to still another aspect of the present invention, there is provided a method of degrading a hard-to-degrade organic compound by using a microorganism capable of degrading the hard-to-degrade organic compound, comprising
a step of proliferating at least one microorganism capable of degrading the hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, in a culture medium containing both a substance capable of inducing the degradation capability of the microorganism and Fe ions, under inorganic conditions; and
a step of controlling the degradation capability of the microorganism by adjusting the concentration of Fe ions in the culture medium.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a graph showing the relation between Fe concentration and proliferation rate of a microorganism in a step of proliferating the microorganism.
FIG. 2 is a graph showing the relation between Fe concentration and microbial degradation rate of cis-1,2-dichloroethylene in a step of proliferating the microorganism.
FIG. 3 is a graph showing the relation between Fe concentration and microbial degradation rate of phenol in a step of proliferating the microorganism.
FIG. 4 is a graph showing the relation between Fe concentration and proliferation rate of a microorganism in a step of proliferating the microorganism.
FIG. 5 is a graph showing the relation between Fe concentration and microbial degradation rate of phenol in a step of proliferating the microorganism.
FIG. 6 is a graph showing the relation between Fe concentration and microbial degradation rate of cis-1,2-dichloroethylene in a step of proliferating the microorganism.
FIG. 7 is a schematic view showing an example of a gas-phase bioreactor to which the method of the present invention can be applied.

### (A method of proliferating a microorganism capable of degrading a hard-to-degrade organic compound)

According to an aspect of the present invention, there is provided a method of proliferating a microorganism capable of degrading a hard-to-degrade organic compound, comprising a step of proliferating the microorganism in a culture medium containing both a substance capable of inducing a degradation capability of the microorganism and Fe ions, under inorganic conditions.

This method makes it possible to sufficiently induce the microbial capability of degrading a hard-to-degrade organic compound, before the microorganism is brought into contact with the organic compound. Therefore, when the microorganism obtained by the method is used for degradation of a hard-to-degrade organic compound, the time required for the microbial degradation can be reduced almost half. Furthermore, by using the method, the proliferation rate of the microorganism can be increased to almost twice.

The term "a hard-to-degrade organic compound" used herein refers to an organic compound which cannot be degraded or is hard to degrade by actions of the natural environment such as soil, air, and rivers. More particularly, "a hard-to-degrade organic compound" refers to an organic halogen compound or an aromatic compound. Furthermore, "a hard-to-degrade organic compound" includes an organic compound used in plastic waste which is specified by the waste disposal law. More specifically, "a hard-to-degrade organic compound" includes organic halogen compounds such as trichloroethylene, cis-dichloroethylene, trans-dichloroethylene, 1,1-dichloroethylene, tetrachloroethylene, dichloroethane, trichloroethane, tetrachloroethane, vinyl chloride, carbon tetrachloride, vinyl fluoride, 3,3,3-trifluoro-2-propene, 2,3-dichlorohexafluoro-2-butene, and vinyl bromide. Also, "a hard-to-degrade organic compound" includes aromatic compounds such as phenol, toluene, xylene, cresol, and benzene; polycyclic aromatic compounds such as naphthalene, biphenyl, and bisphenol A; and halogenated compounds of the polycyclic aromatic compounds such as polychlorinated biphenyl (PCB) and dioxins (including coplanar PCB).

"A microorganism capable of degrading a hard-to-degrade organic compound" to be proliferated in this method may include bacteria, yeasts, fungi, actinomycetes, single-cell algae, and protozoa, which have ability to degrade the aforementioned organic compound. More specifically, "a microorganism capable of degrading a hard-to-degrade organic compound" may be a microorganism capable of degrading the aromatic compound or the organic halogen compound specified above. Examples of such a microorganism are bacteria belonging to the following genera:
*Janibacter* genus including *Janibacter brevis* (e.g., YMCT-001 strain (FERM BP-5282), which has been deposited in Patent Microorganism Depository, National Institute of Bioscience and Human-Technology, Agency of Industrial Science and Technology, and which is described in Jpn. Pat. Appln. KOKAI Publication No. 9-201581 and International Journal of Systematic and Evolutionary Microbiology, No. 50, 1899-1904, 2000, the entire contents of which are incorporated herein by reference);
*Pseudomonas* genus such as *Pseudomonas putida* (e.g., IFO 3538, which is described in The Journal of Biological Chemistry, 258, 2923-2928, 1983, the entire contents of which are incorporated herein by reference), *Pseudomonas cepacia* (e.g., YMCT-003 strain (FERM BP-6085), which is described in Jpn. Pat. Appln. KOKAI Publication No. 11-75826, the entire contents of which are incorporated herein by reference), and *Pseudomonas fluorescens;*
*Rhodococcus* genus such as *Rhodococcus erythropolis* (e.g., IFO 12320, which is described in Applied and Environmental Microbiology, 61, 549-555, 1995, the entire contents of which are incorporated herein by reference);
*Desulfomonile* genus such as *Desulfomonile tiedjei* (e.g., DCB-1, ATCC 49306);
*Bacillus* genus;
*Streptococcus* genus;
*Alcaligenes* genus;
*Acinetobacter* genus;
Achromobacter genus;
*Paracoccus* genus;
*Rhodobacter* genus;
*Rhodobacterium* genus;
*Methylosinus* genus;
*Mycobacterium* genus;
*Nitrosomonas* genus;
*Corynebacterium* genus;
Methanotrophs (i.e., methane-utilizing bacteria); and

the combination of these bacteria. However, "a microorganism capable of degrading a hard-to-degrade organic compound" is not limited to the aforementioned bacteria.

In the present invention, a microorganism is proliferated under inorganic conditions in order to induce its capability of degrading a hard-to-degrade organic compound. The term "inorganic conditions" used herein refers to an environment containing one or more inorganic compounds, typically, inorganic salts. Examples of such inorganic salts include phosphates, nitrates, sulfates, sodium salts, potassium salts, calcium salts, and magnesium salts. The composition of a culture medium containing such inorganic salts is shown, as an example, in Table 1 described later.

In the inorganic conditions, an organic compound may be contained as a sole carbon source for microbial growth. In the present invention, the carbon source may be a substance capable of inducing the microbial degradation capability of a hard-to-degrade organic compound.

A person skilled in the art may choose appropriately conditions for growing a microorganism under inorganic conditions, such as temperature, pH, and composition of a culture medium, depending upon a type of microorganism to be used, a desired proliferation rate, and so on.

In the step of proliferating a microorganism, a microorganism may be proliferated with immobilized to a carrier. Alternatively, a microorganism may not be immobilized to a carrier. When a microorganism is immobilized to a carrier, the microorganism can be protected from the external environment which may shorten life span of the microorganism, such as a toxic substance produced in the middle of the microbial metabolism or a sudden change of temperature. For the reason, generally in this step, it is preferable that a microorganism is immobilized to a carrier.

To immobilize a microorganism to a carrier, for example, a method of adsorbing it onto the carrier or a method of embedding it in the carrier may be used. Examples of the carrier to adsorb a microorganism are nonwoven fabric, woody foamed carrier, silica-based porous ceramics, porous glass, carbon fiber chip, viscose foamed carrier, polyvinyl alcohol, and polyethylene glycol, but not limited to them. Examples of the carrier to embed a microorganism are polymer gels, such as agar, agarose, alginic acid gel, κ-carrageenan, polyacrylamide gel, polyvinyl alcohol gel, and polyethylene glycol, and photo-crosslinkable polymers, but not limited to them.

In the step of proliferating a microorganism, a substance capable of inducing a degradation capability of the microorganism (hereinafter, referred to as "an inducer") is continuously or intermittently added to a culture medium.

In the present invention, the inducer is not particularly limited as long as it can induce the degradation capability of a microorganism. Such an inducer may be a substrate of an enzyme metabolizing an aromatic compound, such as toluene monooxygenase, toluene dioxygenase, phenol hydroxylase, and biphenyl dioxygenase. Specifically, the inducer may be a substrate of an enzyme catalyzing a cleavage reaction of an aromatic ring of an aromatic compound. More specifically, the inducer may be an aromatic compound, and either a monocyclic aromatic compound or a polycyclic aromatic compound. Examples of such an inducer include phenol, toluene, cresol, benzene, 1-bromonaphthalene, bromobenzene, dichlorobenzene, trichlorobenzene, and polychlorinated biphenyl. These aromatic compounds may have a substituent. The substituent may be a general substituent, such as a straight or branched alkyl group, a straight or branched alkylene group, a straight or branched alkynyl group, a hydroxyl group, a halogen group, a carboxyl group, and combination thereof. The inducer may be also a heterocyclic compound containing nitrogen, sulfur, or oxygen. The heterocyclic compound may have the aforementioned substituent(s).

In the case where a microorganism is capable of degrading an organic halogen compound as a hard-to-degrade organic compound, at least one of the aforementioned inducers may be used. Likewise, in the case where a microorganism is capable of degrading an aromatic compound as a hard-to-degrade organic compound, at least one of the aforementioned inducers may be used. In this case, the inducer may be the same as the target compound of degradation.

In the method of the present invention, the inducer may be added in any concentration as long as it can induce the microorganism to degrade a hard-to-degrade organic compound. For example, when an aromatic compound is used as the inducer, the inducer may preferably be added in a concentration of 0.1 mg/L to 10 g/L, more preferably, 1 to 1000 mg/L, and further preferably, 10 to 500 mg/L.

In the step of proliferating a microorganism in the method, Fe ions are added to a culture medium in addition to the aforementioned inducer. As described above, an aromatic compound may be used as the inducer. Most of enzymes metabolizing the aromatic compound are presumed to have an Fe ion at the active center thereof and thus the Fe ion may mediate electron transfer in the enzyme, thereby catalyzing the metabolism of the aromatic compound (see Proteins, Nucleic Acids, and Enzymes, Vol. 45, 1339-1349, 2000, the entire contents of which are incorporated herein by reference). For this reason, when a microorganism is allowed to proliferate in the presence of Fe ions, the microbial capability of degrading the inducer can be increased, with the result that the microbial degradation rate of the hard-to-degrade organic compound defined above, can be also increased.

In the present invention, "Fe ion" may be a free Fe ion, or an Fe ion that is bound or adsorbed to another substance. Typically, "Fe ion" may be a divalent Fe ion or trivalent Fe ion. Alternatively, Fe ion may be added in the form of a complex ion.

It is preferable that the type of Fe ion is a type suitable for the enzyme metabolizing the aforementioned inducer.

Fe ions may be added in a concentration of 0.1 to 100 µg/L, more preferably, 1 to 50µg/L. The concentration of Fe ions may be appropriately determined depending upon the type of microorganism to be used, the microbial degradation rate of a target organic compound, and the microbial metabolism rate of the inducer.

When the concentration of added Fe ions is increased in the aforementioned step, a series of enzymes metabolizing the inducer are induced in a microorganism, with the result that the consumption of the inducer increases. A hard-to-degrade organic compound is degraded by cometabolism of the series of enzymes. Thus, the increase of Fe ion concentration significantly enhances the microbial capability of degrading of the hard-to-degrade organic compound. Therefore, when Fe ions are maintained in appropriate and high concentration while supplying an inducer at appropriate times, the microorganism can maintain a high degradation capability. In addition, in this case, the utilization of the inducer is accelerated, and thereby the proliferation rate of the microorganism increases.

On the other hand, when the concentration of Fe ions is decreased, the microbial capability of degrading a hard-to-degrade organic compound decreases, and at the same time, the consumption amount of an inducer also decreases. Therefore, in this case, the microbial capability of degrading the organic compound can be maintained for a long time, with a small amount of the inducer. This case is advantageous in this respect.

*Janibacter brevis,* which is capable of proliferating by utilizing phenol as a sole carbon source, is taken as an example. When *Janibacter brevis* is allowed to proliferate in a high concentration of Fe ions (e.g., 20 µg/L or more), the rate and capability of degrading phenol increases, and also the capability of degrading a hard-to-degrade organic compound increases. In contrast, when the concentration of Fe ions is low (e.g., 2 µg/L or less), phenol remains for a long time without being degraded. As a result, the degradation capability of *Janibacter brevis* can be continuously induced in the presence of a small amount of phenol.

Similarly in the cases of microorganisms other than *Janibacter brevis,* the microbial capability of degrading both a hard-to-degrade organic compound and an inducer can be appropriately controlled, by adjusting the Fe ion concentration appropriately.

According to the present invention, Fe ions are added besides an inducer in a step of proliferating a microorganism, and thereby it is possible to increase the microbial degradation rate of both a hard-to-degrade organic compound and the inducer as well as the proliferation rate of the microorganism.

According to another aspect, the present invention relates to a method of producing a microorganism having high degradation capability of both a hard-to-degrade organic compound and an inducer, by adding Fe ions besides the inducer in a step of proliferating the microorganism.

According to still another aspect, the present invention relates to a method of manufacturing a carrier to which a microorganism having high degradation capability of both a hard-to-degrade organic compound and an inducer is immobilized, by adding Fe ions besides the inducer in a step of proliferating the microorganism. Also, the present invention relates to a method of producing a bioreactor housing such a carrier.

### (A method of degrading a hard-to-degrade organic compound)

According to another aspect of the present invention, there may be provided a method of degrading a hard-to-degrade organic compound by using a microorganism capable of degrading a hard-to-degrade organic compound, comprising a step of controlling the microbial capability of degrading the hard-to-degrade organic compound by controlling the concentration of Fe ions in a culture medium.

A hard-to-degrade organic compound to be degraded in this method is as described above, and typically, may be a hard-to-degrade organic compound contained in polluted sample such as polluted water, sludge, and polluted soil. Therefore, the method can be used to treat such a polluted sample. According to still another aspect, the method may be said to be a method of purifying such a polluted sample.

In this method, a hard-to-degrade organic compound is degraded by bringing a microorganism capable of degrading the hard-to-degrade organic compound into contact with the hard-to-degrade organic compound. A type of microorganism to be used in the method is as described above.

The microorganism to be used in the method is preferably proliferated in an appropriate medium previously, before brought into contact with a hard-to-degrade organic compound. More preferably, the microorganism to be used in the method is proliferated previously in accordance with the proliferation method described above (i.e., in the presence of both a substance capable of inducing the degradation capability of the microorganism and Fe ions).

Also in this method, the microorganism may be used with immobilized to a carrier. Alternatively, a microorganism may not be immobilized to a carrier. Also in this method, it is preferable that a microorganism is immobilized to a carrier. The carrier for use in immobilizing a microorganism thereto is as described above.

After a microorganism is immobilized to a carrier if necessary, the microorganism-immobilized carrier is directly added to a sample containing a hard-to-degrade organic compound. Alternatively, such a sample is allowed to pass through a bioreactor housing the microorganism-immobilized carrier. In this way, the microorganism is brought into contact with a hard-to-degrade organic compound. An person skilled in the art may choose appropriate conditions for bringing a microorganism into contact with a hard-to-degrade organic compound, such as temperature, pH, and a composition of a culture medium, depending upon a type of microorganism to be used, a desired degradation rate, and so on.

In this method, the aforementioned inducer may be added if necessary. However, the inducer should not be added excessively, since a microorganism proliferates excessively and thus overflows into the wastewater.

In this method, it is necessary to add inorganic salts as basic nutrients in a medium for culturing a microorganism. The culture medium containing inorganic salts is the same as described above. To supply inorganic salts to a microorganism, a solution containing inorganic salts may be periodically supplied to the microorganism. Alternatively, a degradation tank of a bioreactor may be filled with the solution containing inorganic salts.

This method is, in particular, suitable for degrading a hard-to-degrade organic compound by using a bioreactor. In the case where a bioreactor is used, any bioreactor may be used as long as it is a general bioreactor. An example of the general bioreactor may comprises a degradation tank housing a microorganism-immobilized carrier; and further, if necessary, a temperature-controlling device for controlling temperature of the degradation tank; a dispersion means for dispersing the carrier or a polluted sample in the degradation tank; a stirring means for stirring the carrier or the polluted sample; and a circulation system for circulating the polluted sample by taking out it from the degradation tank and returning it to the degradation tank.

FIG. 7 shows a schematic structure of an example of a gas-phase bioreactor. The gas-phase bioreactor has a microorganism-packed tower 1 functioning as a tank for degrading an organic compound. The microorganism-packed tower 1 houses a carrier having a microorganism immobilized thereto, that is, a microorganism-immobilized polyvinyl alcohol (PVA) carrier 2. The temperature of the microorganism-packed tower 1 is controlled by a temperature-controlling device 3. A culture medium stored in a reservoir tank 4 is supplied to the microorganism filled in the microorganism-packed tower 1. The culture medium stored in the reservoir tank 4 is transferred through a circulation pipe 6 with a circulation pump 5, and poured into the microorganism-packed tower 1 from a medium supply port 7. A gaseous hard-to-degrade organic compound is introduced into the microorganism-packed tower 1 from a gas inlet 8, degraded by the microorganism, and discharged from a gas outlet 9.

In this method, the microbial degradation capability is controlled by adjusting the concentration of Fe ions. A type of Fe ions to be used in this method is as described in the aforementioned proliferation method.

As described above, when added Fe ion amount is increased in microbial degradation of an organic compound, the microbial degradation capability of both the organic compound and an inducer increases, and also the proliferation rate of the microorganism increases. In general, it is not necessary for a microorganism to proliferate quickly in the step of degrading an organic compound. Therefore, in most cases in the step of degrading an organic compound, Fe ion concentration is preferably set lower than that in the step of starting up a bioreactor by preparing a microorganism. For example, when *Janibacter brevis* is used and the Fe ion concentration is reduced to as low as 2 µg/L, it is possible to suppress a microorganism from excessively proliferating and from excessively consuming an inducer. As a result, a bioreactor can be operated at a sufficient degradation rate while maintaining a sufficient amount of microorganism.

In the case where the microbial degradation capability decreases in some reason, Fe ion concentration is preferably increased. In this case, it is also useful that an inducer may be newly supplied to a medium. The fact that a microbial degradation capability decreases can be know, based on an increase of the amount of a carbon source remaining in the medium and/or a decrease of the existing amount of the microorganisms. On the other hand, it is possible to know the fact that the microbial degradation capability is excessively high, based on a decrease of the amount of a carbon source remaining in the medium and/or an increase of the existing amount of the microorganisms.

As described above, Fe ion concentration is controlled in accordance with the method of the present invention, and thereby it is possible to control the microbial capability of degrading an organic compound and an inducer as well as the proliferation rate of the microorganism.

The method of the present invention is also applied to a method of degrading an organic compound or an inducer discharged into wastewater from a bioreactor. According to the present invention, the organic compound or the inducer discharged into wastewater from a bioreactor is degraded in control of the microbial degradation capability of the organic compound or the inducer as well as the proliferation rate of the microorganism by adjusting the concentration of Fe ions. When Fe ions are added to the wastewater in a concentration of e.g., 40 µg/L, the microbial degradation rate of the organic compound or the inducer can be accelerated.

The present invention will be described in more detail by way of examples. However, the present invention is not limited to the examples.

### (Example 1)

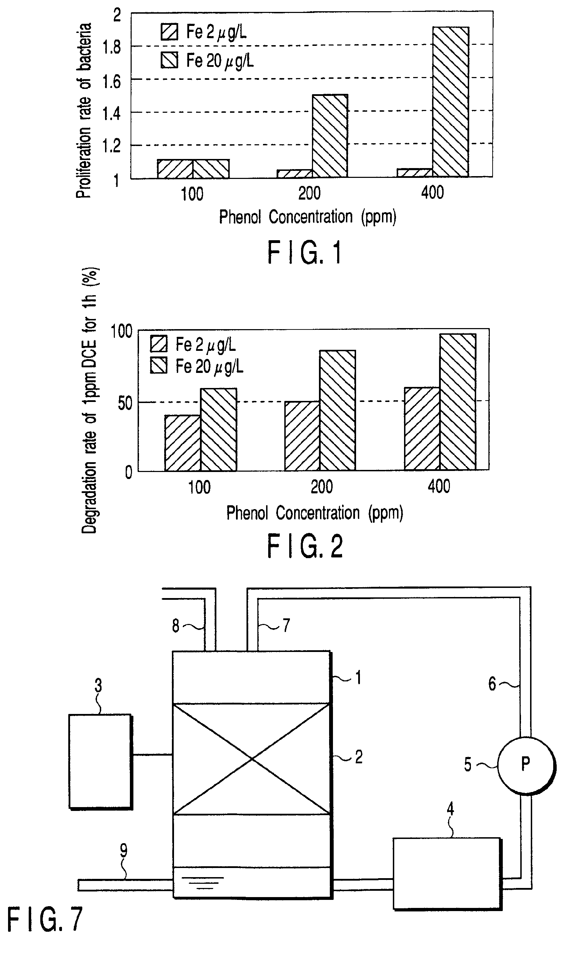

In this example, the method of proliferating a microorganism of the present invention and characteristics of the microorganism that is obtained by the method will be described referring to FIGS. 1 and 2.

*Janibacter brevis* that was cultured in a nutrient medium previously was suspended in 15 mL of inorganic salt medium (Table 1) so as to obtain an optical density (OD) of 1 at the wavelength of 660 nm.

**Table 1**

| | |
|---|---|
| KH₂PO₄ | 1.7g/L |
| Na₂HPO₄ | 9.8g/L |
| (NH₄)₂SO₄ | 1.0g/L |
| MgSO₄·7H₂O | 0.1g/L |
| MgO | 10.75 mg/L |
| CaCO₃ | 2.00 mg/L |
| ZnSO₄·7H₂O | 1.44 mg/L |
| CuSO₄·5H₂O | 0.25 mg/L |
| CoSO₄·7H₂O | 0.28 mg/L |
| H₃BO | 0.06 mg/L |
| HCl | 51.3 µL |
| | |
| pH 7.3 | |

Phenol was added in a concentration of 100, 200, or 400 ppm to the inorganic salt medium containing *Janibacter brevis.* FeSO₄·7H₂O was also added. It was cultured with shaking at 25°C. Test was performed for two types of FeSO₄·7H₂O concentration, 0.01 mg/L and 0.1 mg/L (i.e., Fe ion concentration of 2 µg/L and 20 µg/L) with respect to two types of phenol concentration, respectively.

The bacteria allowed to proliferate in the aforementioned medium for 24 hours. The proliferation rate of the bacteria and their degradation capability of an organic chlorine compound are shown in FIGS. 1 and 2. In FIG. 1, the proliferation rate of the bacteria is shown by the ratio of the bacteria amount after proliferation (i.e., 24 hours after the initiation of the culture) to the bacteria amount before proliferation.

As shown in FIG. 1, when Fe ion concentration was low (2 µg/L), no significant increase was observed in the proliferation rate of the bacteria even if phenol concentration (i.e., inducer concentration) increased. In contrast, when Fe ion concentration was high (20 µg/L), the proliferation rate of the bacteria increased significantly as phenol concentration increased. The proliferation rate of the bacteria increased at most twice.

The microbial degradation capability of an organic chlorine compound was evaluated as follows. First, a suspension of the bacteria was prepared by suspending the bacteria in an inorganic salt medium so as to have an OD of 0.2 at 660 nm (OD 660 nm = 0.2). 10 mL of the bacteria suspension was placed in a 25 mL vial, and the vial was capped tight. Thereafter, 1 ppm of cis-1,2-dichloroethylene (DCE) was added to the vial. The vial was incubated with stirring at 25°C for one hour. The amount of DCE remaining in a headspace of the vial was measured with GC-PID.

As is apparent from FIG. 2, when the bacteria are allowed to proliferate in the presence of a low concentration of Fe ions (2 µg/L), the bacteria exhibited almost a constant degradation rate of DCE regardless of phenol concentration. In contrast, when the bacteria are allowed to proliferate in the presence of a high concentration of Fe ions (20 µg/L), the bacteria exhibited a high degradation rate of DCE as phenol concentration increased. The bacteria that were proliferated in the medium containing Fe ions and phenol both in a high concentration exhibited a degradation rate almost twice as large as the bacteria that were proliferated in the medium containing Fe ions and phenol both in a low concentration.

As is clear from this example, when bacteria proliferate in a medium containing a high concentration of Fe ions in the presence of an inducer, it is possible to obtain a microorganism having an enhanced proliferation rate and high degradation capability of an organic compound.

### (Example 2)

In this example, the microbial proliferation rate in the case where a microorganism is immobilized to a carrier will be explained. In this case, the microorganism immobilized to a carrier is proliferated in accordance with the method of the present invention.

*Janibacter brevis* was embedded in a polyvinyl alcohol gel, thereby preparing a microorganism-immobilized carrier. To describe more specifically, the microbial cells were mixed with an aqueous solution of 12% polyvinyl alcohol so as to have an OD of 0.5 at 660 nm. The mixture was spread on a tray and frozen. It was left still overnight, and thereafter gradually thawed at room temperature.

After a freeze-thaw cycle was repeated three times, the gel was cut into pieces of 5 mm squares. 75 mL of the gel (that is, the microorganism-immobilized carrier) was added to 75 mL of inorganic salt medium. Further 100 or 200 ppm of phenol was added to the medium. FeSO₄·7H₂O was also added. It was incubated with shaking at 25°C. Test was performed for two types of FeSO₄·7H₂O concentration, 0.01 mg/L and 0.1 mg/L (i.e., 2 µg/L and 20 µg/L of Fe ion concentration) with respect to two types of phenol concentration, respectively.

Since phenol was completely consumed in samples containing a higher concentration of Fe ions (20 µg/L) on one day after the incubation started, additional phenol was added in an amount twice as large as that added on the previous day, and the shaking culture was continued. Such a operation was repeated for 4 days, and added phenol amount was increased day by day. As a result, the microbial degradation capability of an organic chlorine compound reached saturation after 4 days. On the other hand, in the cases of samples containing a lower concentration of Fe ions (2 µg/L), phenol was completely consumed in a sample containing 100 ppm of phenol, whereas phenol still remained in a sample containing 200 ppm of phenol. Therefore, the sample containing 100 ppm of phenol was continuously cultured while the added phenol amount was increased by 100 ppm per day. As a result, the microbial degradation capability of an organic chlorine compound reached saturation after two weeks.

The reason the degradation capability reached saturation is that the microbial cells immobilized to the carrier proliferated and thereby reached saturation.

From the foregoing, it is clear that the proliferation rate of a microorganism immobilized to a carrier can be increased three-fold or more according to the method of the present invention. It is therefore demonstrated that the method of the present invention is very useful in rapidly starting up a bioreactor using a microorganism-immobilized carrier.

### (Example 3)

In this example, the relation between Fe ion concentration and phenol degradation rate, the relation between Fe ion concentration and bacteria proliferation rate, and the relation between Fe ion concentration and capability of degrading an organic chlorine compound in a step of proliferating the bacteria were investigated.

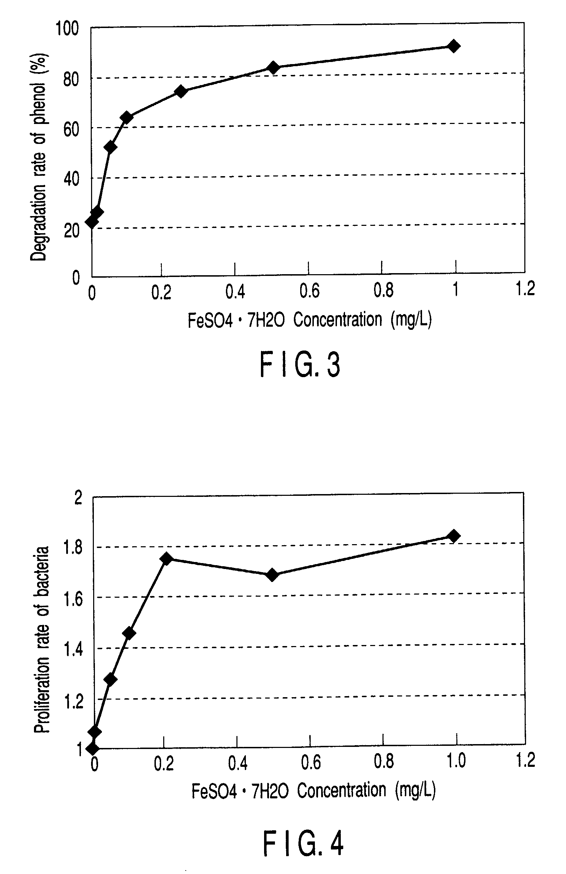

*Janibacter brevis* that was cultured in a nutrient medium previously was suspended in 10 mL of inorganic salt medium (Table 1) so as to obtain an OD of 1 at 660 nm. Phenol was added in a concentration of 100 ppm to the inorganic salt medium containing *Janibacter brevis.* FeSO₄·7H₂O was added to prepare samples having FeSO₄·7H₂O concentration of 0, 0.01, 0.05, 0.1, 0.25, 0.5 or 1 mg/L. The relation between Fe ion concentration and phenol degradation rate (at 5 hours after the initiation of the degradation) was investigated for the samples. FIG. 3 shows the phenol degradation rate at 5 hours after the initiation of the degradation.

It is apparent from FIG. 3 that the phenol degradation rate increases with an increase of Fe ion concentration.

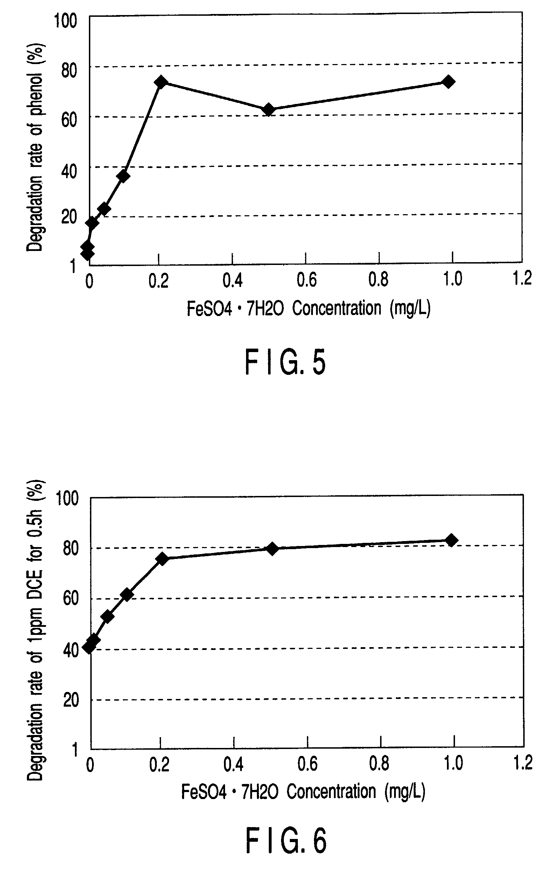

Next, *Janibacter brevis* that was cultured in a nutrient medium previously was suspended in 20 mL of inorganic salt medium (Table 1) so as to obtain an OD of 1 at 660 nm. Phenol was added in a concentration of 400 ppm to the inorganic salt medium containing *Janibacter brevis.* FeSO₄·7H₂O was added to prepare samples having FeSO₄·7H₂O concentration of 0, 0.001, 0.01, 0.05, 0.1, 0.2, 0.5 or 1 mg/L. The samples were cultured at 25°C with shaking. The proliferation rate of the bacteria and the phenol degradation rate on one day after the initiation of the culture are shown in FIG. 4 and FIG. 5, respectively. In FIG. 4, the proliferation rate of the bacteria is shown by the ratio of the bacteria amount after the culture (i.e., one day after the initiation of the culture) to the bacteria amount before the culture. The proliferation rate and phenol degradation rate significantly increased with an increase of Fe ion concentration. Both the proliferation rate and phenol degradation rate were almost constant at 0.2 mg/L or more of FeSO₄·7H₂O concentration.

Furthermore, the degradation capability of an organic chlorine compound was investigated based on the degradation rate of 1 ppm of cis-1,2-dichloroethylene. The test was performed using a 25 mL vial and 10 mL of bacteria-suspended medium with an OD of 0.2 at 660 nm. FIG. 6 shows the degradation rate at 0.5 hours after the initiation of the incubation. The degradation rate increased with an increase of Fe ion concentration, and reached almost constant at 0.2 mg/L or more of FeSO₄·7H₂O concentration.

As is apparent from this example, it is possible to give a microorganism a desired proliferation rate, desired degradation capability of an organic compound, and desired degradation capability of an inducer, by adjusting Fe ion concentration appropriately during proliferation.

### (Example 4)

In this example, a procedure for rapidly starting up a bioreactor according to the method of the present invention will be explained.

70 L of gas-phase bioreactor (FIG. 7) was packed with polyvinyl alcohol (PVA) carrier prepared by a freeze-thaw method (see Example 2). *Janibacter brevis* was embedded in the carrier as a microorganism for degrading an organic compound.

The microorganism-packed tower 1 was a cylinder of 180 cm height and 30 cm diameter. The microorganism-packed tower 1 was packed with microorganism-immobilized PVA carrier 2 up to 110 cm in height. As the PVA carrier 2, 12 mm squares of PVA gel pieces were used. To allow the microorganism to sufficiently proliferate within the carrier, an inorganic salt medium containing phenol was supplied from the top of the microorganism-packed tower 1 like a shower. The medium was sprayed at a flow rate of 2.5 L/minute, twice a day, for 30 minutes per time. The concentration of phenol was set at 400 ppm.

The performance of the reactor was evaluated based on a degradation rate (outlet gas concentration/inlet gas concentration) of 250 ppmV of cis-1,2-dichloroethylene gas having 2 cm/minute of superficial velocity. When a FeSO₄·7H₂O concentration in an inorganic salt medium to be supplied was 0.01 mg/L, the degradation rate reached a constant value (80%) after two weeks. When a FeSO₄·7H₂O concentration was 1 mg/L, the degradation rate reached a constant value (95%) after 4 days.

In this example, it is demonstrated that the time to start up a bioreactor can be reduced by setting Fe ion concentration to be high. Additionally, it is demonstrated that the microbial degradation rate of an organic compound can be maintained at a high rate by setting Fe ion concentration to be high.

### (Example 5)

In this example, the steady operation of a bioreactor started up according to the method of Example 4 will be described.

When the bioreactor was operated maintaining 1 mg/L of FeSO₄·7H₂O concentration and 400 ppm of phenol concentration in the same way as in the starting-up time, the bacteria proliferated excessively and overflowed from the carrier. Masses of the overflowed bacteria (i.e., scum) were formed on the bottom of the reactor and in a circulating liquid, with the result that it interfered with a continuous operation of the bioreactor.

The scum was removed, and then the operation was continued reducing the FeSO₄·7H₂O concentration to 0.01 mg/L and the phenol concentration to 200 ppm. Thereby, the excessive proliferation of the bacteria did not occur, and the amount of the scum reduced to a little. As a result, the operation was performed without interference.

As is clear from this example, the proliferation rate of the microorganism can be appropriately controlled during the microbial degradation step of an organic compound, by adjusting Fe ion concentration according to the method of the present invention.

### (Example 6)

In this example, a method of degrading phenol and cis-1,2-dichloroethylene overflowing into wastewater without degrading will be explained.

In the aforementioned bioreactor, an inorganic salt medium containing phenol was supplied like a shower to a microorganism twice a day, and the medium was allowed to circulate and reused. Since inorganic salts contained in the circulating medium were gradually consumed, the circulating medium was exchanged with a fresh medium twice a month. When steady operation of the bioreactor was continued in a FeSO₄·7H₂O concentration of 0.01 mg/L, undegraded phenol and undegraded cis-1,2-dichloroethylene remained in the wastewater.

The wastewater was taken out, and FeSO₄·7H₂O was added to it to bring its concentration to 0.5 mg/L or more. It was left still, and then the microorganism contained in the wastewater increased the degradation capability. Both phenol and cis-1,2-dichloroethylene were degraded up to or below the lowermost detection limit after 2 days. On the other hand, when the wastewater was heated to about 30°C, the degradation time was further reduced, and both phenol and cis-1,2-dichloroethylene were not detected on the following day.

After confirming that phenol and cis-1,2-dichloroethylene were not detected, NaOH was added to the wastewater to bring pH to 9 or more, and it was left still overnight. As a result, no living bacteria were observed in the wastewater. This fact means that the bacteria cells were destroyed with alkali.

From the foregoing, the following facts are found. When a microorganism is contained in wastewater, the microbial degradation rate of an inducer and an organic compound contained in the wastewater can be increased by adding Fe ions to the wastewater. After finishing the degradation of the inducer and organic compound, the remaining microorganism can be easily destroyed if alkali is added to the wastewater. Accordingly, the method of this example is extremely effective in bringing microbial purification of the environment closer to perfection.

### (Example 7)

In this example, Fe ions were added to various samples having variety in both bacteria species and target compounds of degradation, and the effects of Fe ions were examined. The "initial Fe concentration" in each of the samples was set at a constant concentration (i.e., 0.01 mg/L of FeSO₄·7H₂O). After that, Fe ions were added, and the "Fe concentration after addition" in each of the samples was appropriately set as shown in Tables 2 to 4. The degradation amounts of the target compound per hour are compared between the time of the "initial Fe concentration" and the time of "Fe concentration after addition". The degradation amounts at the time of the "initial Fe concentration" is shown in Tables 2 to 4 in the columns of "Initial degradation amount". The degradation amount at the time of "Fe concentration after addition" is shown in Tables 2 to 4 in the columns of "Degradation amount after addition". As is apparent from the tables, the degradation amount of each sample increased by addition of Fe ions. However, when Fe ion concentration is excessively high (100 mg/L), it has little effect upon the degradation amount (See Example B-5).

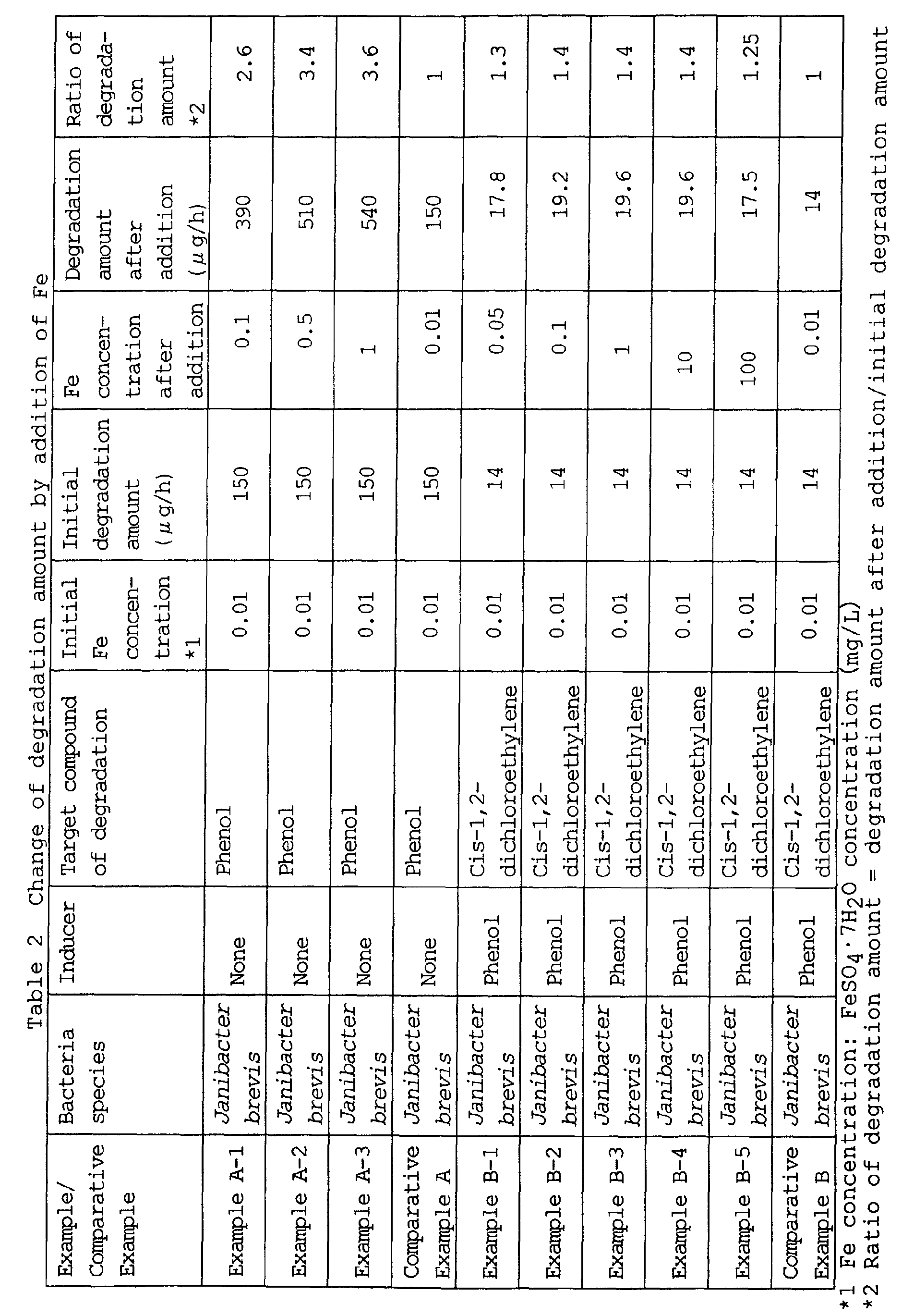

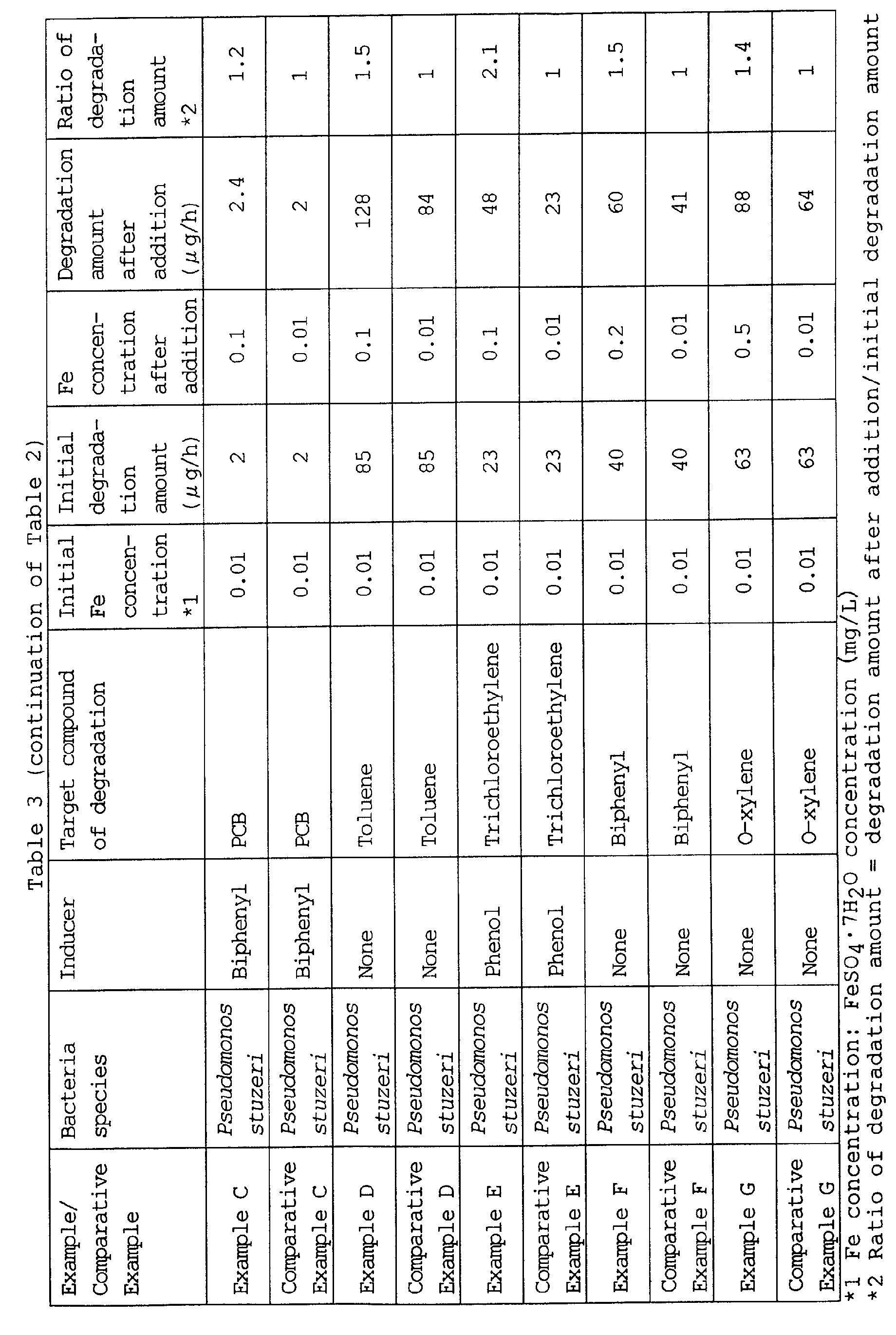

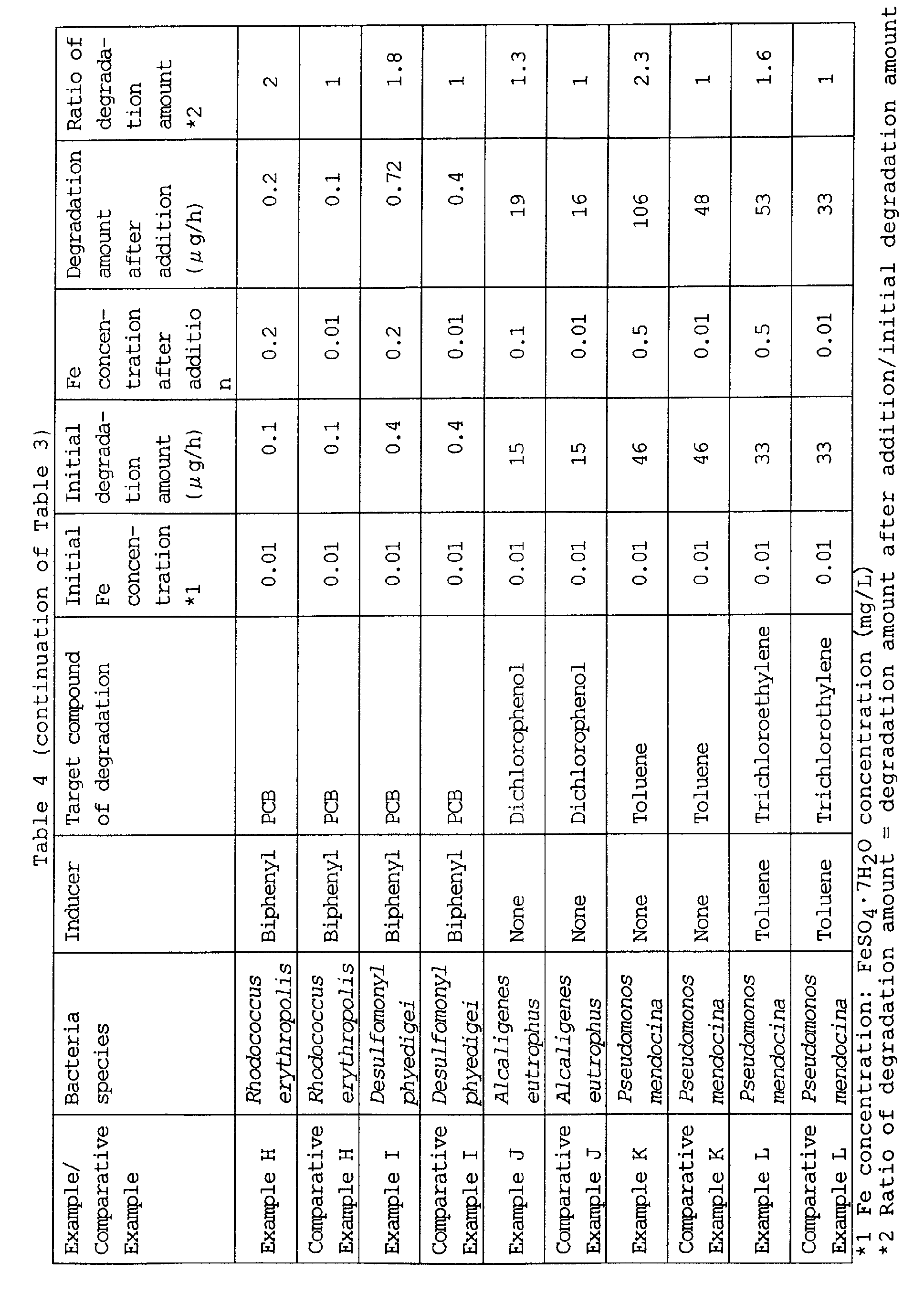

The method of the present invention makes it possible to increase the proliferation rate of a microorganism and the microbial degradation rate of an organic compound. As a result, the microbial degradation treatment of an organic compound can be accelerated, and the treatment can be completed in a shorter time.

Furthermore, the method of the present invention makes it possible to artificially control the degradation of an organic compound.

## Claims

1. A method of proliferating a microorganism capable of degrading a hard-to-degrade organic compound, **characterized by** comprising:
proliferating at least one microorganism capable of degrading a hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, in a culture medium containing both a substance capable of inducing the degradation capability of the microorganism and Fe ions, under inorganic conditions.

2. A method according to claim 1, **characterized in that** the microorganism is at least one selected from the group consisting of *Janibacter brevis, Pseudomonas putida, Pseudomonas cepacia, Pseudomonas fluorescens, Pseudomonas stuzeri, Rhodococcus erythropolis, Desulfomonile tiedjei, Alcaligenes eutrophus,* and *Pseudomonas mendocina.*

3. A method of degrading a hard-to-degrade organic compound by using a microorganism capable of degrading the hard-to-degrade organic compound, **characterized by** comprising:
controlling the degradation capability of at least one microorganism capable of degrading the hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, by adjusting the concentration of Fe ions in a culture medium.

4. A method according to claim 3, **characterized in that** the microorganism is at least one selected from the group consisting of *Janibacter brevis, Pseudomonas putida, Pseudomonas cepacia, Pseudomonas fluorescens, Pseudomonas stuzeri, Rhodococcus erythropolis, Desulfomonile tiedjei, Alcaligenes eutrophus,* and *Pseudomonas mendocina.*

5. A method of degrading a hard-to-degrade organic compound by using a microorganism capable of degrading the hard-to-degrade organic compound, **characterized by** comprising:
proliferating at least one microorganism capable of degrading the hard-to-degrade organic compound selected from the group consisting of *Janibacter* genus, *Pseudomonas* genus, *Rhodococcus* genus, *Desulfomonile* genus, *Alcaligenes* genus, *Bacillus* genus, *Streptococcus* genus, *Acinetobacter* genus, *Achromobacter* genus, *Paracoccus* genus, *Rhodobacter* genus, *Rhodobacterium* genus, *Methylosinus* genus, *Mycobacterium* genus, *Nitrosomonas* genus, *Corynebacterium* genus, and Methanotrophs, in a culture medium containing both a substance capable of inducing the degradation capability of the microorganism and Fe ions, under inorganic conditions; and
controlling the degradation capability of the microorganism by adjusting the concentration of Fe ions in the culture medium.

6. A method according to claim 5, **characterized in that** the microorganism is at least one selected from the group consisting of *Janibacter brevis, Pseudomonas putida, Pseudomonas cepacia, Pseudomonas fluorescens, Pseudomonas stuzeri, Rhodococcus erythropolis, Desulfomonile tiedjei, Alcaligenes eutrophus,* and *Pseudomonas mendocina.*

7. A method according to claim 1, **characterized in that** said substance capable of inducing the degradation capability of the microorganism is an aromatic compound.

8. A method according to claim 2, **characterized in that** said substance capable of inducing the degradation capability of the microorganism is an aromatic compound.

9. A method according to claim 5, **characterized in that** said substance capable of inducing the degradation capability of the microorganism is an aromatic compound.

10. A method according to claim 6, **characterized in that** said substance capable of inducing the degradation capability of the microorganism is an aromatic compound.

11. A method according to claim 7, **characterized in that** a concentration of said aromatic compound is 0.1 to 10,000 mg/L and a concentration of said Fe ions is 0.1 to 100 µg/L.

12. A method according to claim 8, **characterized in that** a concentration of said aromatic compound is 0.1 to 10,000 mg/L and a concentration of said Fe ions is 0.1 to 100 µg/L.

13. A method according to claim 9, **characterized in that** a concentration of said aromatic compound is 0.1 to 10,000 mg/L and a concentration of said Fe ions is 0.1 to 100 µg/L.

14. A method according to claim 10, **characterized in that** a concentration of said aromatic compound is 0.1 to 10,000 mg/L and a concentration of said Fe ions is 0.1 to 100 µg/L.

15. A method according to claim 3, **characterized in that** a concentration of said Fe ions is controlled based on an amount of a carbon source remaining in the culture medium and/or an amount of the microorganism capable of degrading the hard-to-degrade organic compound.

16. A method according to claim 4, **characterized in that** a concentration of said Fe ions is controlled based on an amount of a carbon source remaining in the culture medium and/or an amount of the microorganism capable of degrading the hard-to-degrade organic compound.

17. A method according to claim 5, **characterized in that** a concentration of said Fe ions is controlled based on an amount of a carbon source remaining in the culture medium and/or an amount of the microorganism capable of degrading the hard-to-degrade organic compound.

18. A method according to claim 6, **characterized in that** a concentration of said Fe ions is controlled based on an amount of a carbon source remaining in the culture medium and/or an amount of the microorganism capable of degrading the hard-to-degrade organic compound.

19. A method according to claim 9, **characterized in that** a concentration of said Fe ions is controlled based on an amount of a carbon source remaining in the culture medium and/or an amount of the microorganism capable of degrading the hard-to-degrade organic compound.

20. A method according to claim 10, **characterized in that** a concentration of said Fe ions is controlled based on an amount of a carbon source remaining in the culture medium and/or an amount of the microorganism capable of degrading the hard-to-degrade organic compound.
